# EUROPEAN PATENT APPLICATION

(11) **EP 1 389 458 A1**
(43) Date of publication of application: **18.02.2004**
(21) Application number: 01981062.1
(22) Date of filing: 09.11.2001
(51) Int. Cl.: A61H 35/04

(54) **MEDICAL RINSING AND SUCKING DEVICE**

(30) Priority: 27.04.2001 JP 2001133562
(71) Applicant: Nagashima Medical Instruments Co., Ltd, Chiyoda-ku, Tokyo 113-0033 (JP)
(72) Inventor: HOSODA, Yasuo, Tanaka Bldg. 2nd Floor, Toyonaka-shi, Osaka 560-0881 (JP)
(74) Representative: Rackham, Stephen Neil
(86) International application number: PCT/JP2001/009845
(87) International publication number: WO 2002/089722

(57) **Abstract**

The present invention comprises the connection tube (10), having a basal end portion connected with the waste matter sucking tube (44) communicating with the waster matter tank provided with the sucking pump (30), and a jet nozzle (19) for jetting the rinsing water, having a basal end portion connected with a rinsing water tube 30 communicating with a rinsing water tank (33) provided with a compressor (31), and characterized by that the connection tube (10) and the jet nozzle (19) are installed with their respective end portions are installed orienting the same directions, so that the operation for changing the types of the rostral sucking nozzle 12 or the repetitive alternate operation for the jetting of the rinsing liquid and the operation for the drawing of the waste water resulting from the rinsing operation or the simultaneous operation for the both can be simplified. The present device is specially suited for the treatment or the surgical operation for the narrow and deep affected part such as those located in the nasal cavity, interior of the ear and the interior of the throat.

## Description

### TECHNICAL FIELD

The present invention relates to a medical rinsing and sucking device applicable to the surgical operation and treatment of the affected part located specially deep and narrow parts of the bodily organs of the patient suffering from the ear, nose or throat disease.

### BACKGROUND ART

The rinsing following the ear disease treatment is expected to be effective not only for the sterilizing effect but also for the painless removal of earwax and dried scab. The rinsing incidental to the surgical operation in the ear disease treatment serves various purposes and effects such as the rinsing of the area subjected to the surgical operation, the prompt aid for the stopping of the bleeding, the rinsing of the endoscope lens in use and so on.

In conventional method of the rinsing during the surgical operation of the treatment for the ear disease, a rinsing liquid jet nozzle is held in one hand of an operator to jet the rinsing liquid against the affected part for the rinsing thereof and then the jet nozzle is replaced with a rostral sucking nozzle to remove the waste water.

The conventional rinsing method requires some cumbersome processes such as the switching between the jet nozzle for the rinsing liquid and the rostral sucking nozzle for the waste water and the corresponding switching from the compressor to the sucking pump and vice versa.

Further, it has been desired for the conventional rinsing and sucking device to be designed for permitting the quick replacement among a plurality of rostral sucking nozzles not only for avoiding the use of the same sucking nozzle for different patients but also depending on the conditions of the individual patients differing in the size (width and length) and the depth (shallow or deep) and the conditions of individual sucking nozzles such as the clogging with the solids included in the waste water or the degree of deterioration owing to the corrosion.

An object of the present invention is to provide a medical rinsing and sucking device designed for enabling an operator to carry out the single-handed operations such as the jetting of the rinsing liquid, drawing of the waste water resulting from the rinsing and excision of the protrusion of an affected.

Another object of the present invention is to provide a medical rinsing and sucking device not only being excellent in operability but also permitting the operator to replace the sucking nozzle quickly depending on the uses and change in the direction of the sucking hole depending on the location of the affected part.

Further another object of the present invention is to provide a device designed suitably for the surgical operation or the treatment of the affected part being small in width and large in depth in the ear, nose or throat disease treatment.

Further another object of the present invention is to provide a device being capable of simultaneously carrying out the treatment and the rinsing of the affected part and the rinsing of the front end of the photoconductive rod while observing the affected part through a nasal cavity endoscope.

### DISCLOSURE OF THE INVENTION

The present invention comprises a connection tube 10, having the basal end portion connected with a waste matter sucking tube 44 communicating with a waste tank 29 provided with a sucking pump 30 and the front end connected with a rostral sucking tube 12 for drswing the waste water, a jet nozzle 19 for jetting the rinsing water, having the basal end portion connected with a rinsing liquid tube 20 communicating with the rinsing water tank 33 provided with a compressor, and is characterized by that the connection tube 10 and the jet nozzle 19 are installed so that the respective front ends thereof are oriented to substantially the same direction. Hence, the repetitive alternate operations for the jetting of the rinsing liquid and the following waste water drawing, the simultaneous operations for the both and the operation for either one of the both can be simplified. The present invention is particularly suits the application to the surgical operation or the treatment of the narrow and deep affected part in the ear, nose or throat.

In the present invention, the rostral sucking nozzle 12 is detachably connected with the front end of the connection tube 10. Thus, when a plurality of the rostral sucking nozzles 12 varying in the shape of the rostrum are made available, any one particular rostral sucking nozzle can be replaced quickly with another depending on the purposes of their uses.

In the present invention, a sucking control chamber 16 is interposed between the connection tube 10 and the waste matter sucking tube 44; an end portion for insertion 43 is detachably connected with the basal portion for a connecting base 41 of the sucking control chamber 16; the sucking control chamber 16 is integrally formed with an injection valve 22 being provided with an intermediate push button 21 for opening and closing the rinsing liquid tube 20 being connected with the jet nozzle 19.

Thus, it is possible for an operator to start and stop the jetting of the rinsing water 17 and control the sucking, the stop of the sucking of the waste water 18 and the control of the sucking rate by single-handed operation.

### BRIEF DESCRIPTION OF THE DRAWINGS

Fig. 1 (a) is a plan of the principal parts of the medical rinsing and sucking device for the right-handers according to the present invention, while Fig. 1 (b) is a front view of the same.
Fig. 2 is a plan of the principal parts of the medical rinsing and sucking device for the left-handers according to the present invention.
Fig. 3 (a), (b) (c) and (d) are the perspective views of the rostral sucking nozzles 12 differing in the form of the front end 14 thereof.
Fig. 4 (a), (b), (c), (d) and (e) are the perspective views of the jet nozzles 19 varying in front end construction.
Fig. 5 shows another embodiment of the jet valve 22, wherein (a) is a sectional view of the jet valve 22 on line C-C, while (b) is a sectional view of the jet valve 22 on line D-D.
Fig. 6 is a general view of the medical rinsing and sucking device according to the present invention.
Fig. 7 (a) is a sectional view of an embodiment, wherein the storage bag 63 containing the rinsing water 17 is directly pressurized, while Fig. 7 (b) is a sectional view of another embodiment, wherein the storage bag 63 containing the rinsing water 17 is pressurized through an internal bag 69.
Fig. 8 (a) is a front view of the medical rinsing and sucking device as another embodiment of the present invention; (b) shows the sectional views of a sucking needle 71 and a jet needle 70 differing in the front end form; (c) is a front view of an embodiment wherein the sucking needle 71 is connected with an injection cylinder 72 serving as a sucking pump.
Fig. 9 (a) a partially cutaway front view of the medical rinsing and sucking device according to the present invention for application to one nasal cavity, while (b) is a sectional view of the same taken on line E-E.
Fig. 10 is a partially cutaway front view of the medical rinsing and sucking device according to the present invention designed for application to two nasal cavities.
Fig. 11 (a) is a partially cutaway front view of the medical rinsing and sucking device according to the present invention attached to a rhinoscope 59, while (b) is a sectional view taken on line F-F, and (c) is a sectional view of a different embodiment taken on line F-F.
Fig. 12 (a) is a partially cutaway front view of a mechanism designed for excising the protrusion of the affected part by moving back and forth an externally installed sliding cylinder 74 of the rostral sucking nozzle 12; (b) is an enlarged view of the principal parts of the mechanism given in (a); (c) is a front view of the mechanism shown in (a) attached to the sucking control chamber 16.
Fig. 13 (a) is a partially cutaway front view of a mechanism designed for excising the protrusion by the cutter blade 86 provided inside the rostral sucking nozzle 12 and to be driven by a motor 83, while (b) is an enlarged view of the principal parts of the mechanism shown in (a).
Fig. 14 (a) is a sectional view taken on line H-H of another embodiment of the sucking control chamber 16 given in (b); (b) is a sectional view taken on line I-I of (a); (c) is an exploded perspective view of a first pushing member 88 and a second pushing member 89; (d) is a perspective view of the assembled sucking control chamber 16.
Fig. 15 is a perspective exploded view of a sucking control chamber 16 as another embodiment.
Fig. 16 (a) is a sectional view taken on the line along the through hole 47 of the assembled sucking control chamber 16 shown in Fig. 15 and a sectional view of the filter container 104 to be connected with the sucking control chamber 16; (b) is a sectional view of the central portion of the sucking control chamber 16; (c) is a sectional view of an embodiment, wherein the position of the front end and the position of the rear end of a swinging member 50 are reversed, taken on the sectional line including the through hole 47 of the assembled sucking control chamber 16.
Fig. 17 is a partially cutaway front view of a mechanism designed for excising the protrusion by moving back and forth the rostral sucking nozzle 12 installed inside the sliding cylinder 74.
Fig. 18 is a partially cutaway front view of an embodiment of the present invention wherein the medical rinsing and sucking device is designed into a scissors from.
Fig. 19 is a partially cutaway back view corresponding to the view given in Fig. 18.
Fig. 20 (a) is a sectional view of the principal parts of the mechanism for excising the protrusion by rotating the cutter blade 86, provided at the front end of the rostral sucking nozzle 12, with a motor 83, and (b) is the front view of the device shown in (a).
Fig. 21 is a partially cutaway view of an embodiment improved from the embodiment shown in Fig. 18.
Fig. 22 is a perspective exploded view of the principal parts shown in Fig. 21; (a) is an embodiment wherein a sucking hole 80 is set upward; (b) is an embodiment wherein the sucking hole 80 is set downward; (c) is an embodiment wherein the sucking hole 80 is set leftward; (d) is an embodiment wherein the sucking hole 80 is set rightward.
Fig. 23 is a sectional view of the principal parts shown in Fig. 21.
Fig. 24 is a sectional view of the sucking control chamber 16 given in Fig. 21.
Fig. 25 (a), (b), (c), (d), (e) and (f) are partially cutaway enlarged views of the sucking holes 80 differing in form.
Fig. 26 (a), (b) and (c) are front views of the rostral sucking nozzles 12 having curved front portions differing in form.

### BEST MODE FOR CARRYING OUT THE INVENTION

The embodiments of the medical rinsing and sucking device according the present invention will be described below referring to the pertinent drawings.

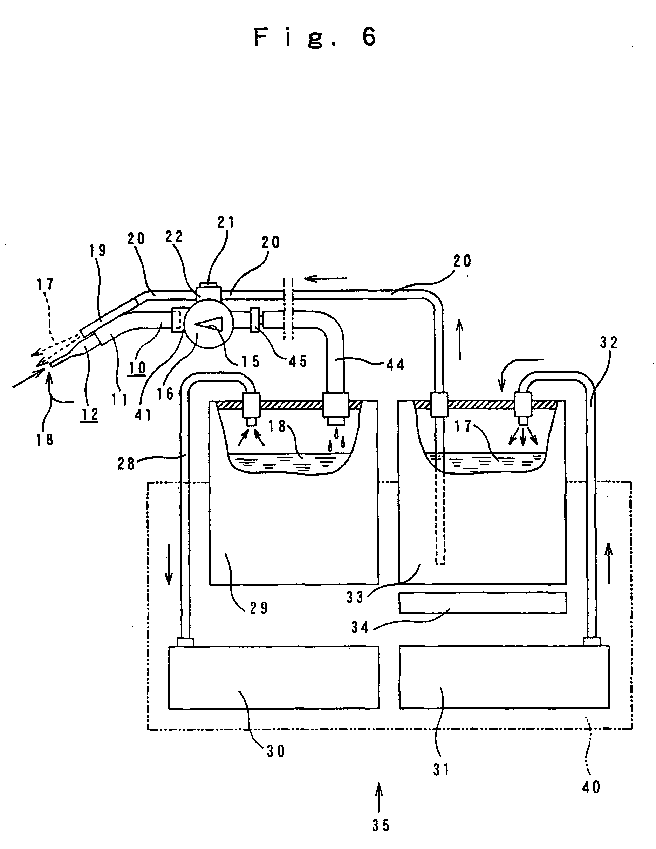

As shown in Fig. 6, a casing 40, constituting the rinsing and sucking unit 35, contains a sucking pump 30 and a compressor 31, while a waste matter tank 29 and a rinsing water tank 33 are installed so as to be removable from above the casing 40. Of these components, the sucking pump 30 and the waste matter tank 29 are connected with each other through a sucking tube 28, while the compressor 31 and the rinsing water tank 33 are connected with each other through a pressurizing tube 32. Provided under the rinsing water tank 33 is a heater 34 for heating the rinsing water to the level equal to the man's body temperature.

The waste matter tank 29 is connected with a waste matter sucking tube 44 for drawing the waste water 18 besides the sucking tube 28, while the rinsing water tank 33 is connected with a rinsing liquid tube 20 for supplying the rinsing water 17 besides the pressurizing liquid tube 32.

Before the front end of the waste matter sucking tube 44, the basal portion of the sucking control chamber 16 is detachably connected with the connection tube 10; further, the rostral sucking nozzle 12 is detachably connected with the connection tube 10; the jet nozzle 19 is connected with the rinsing liquid tube 20 through the interposed jet valve 22, the jet nozzle 19 is integrally installed with the connection tube 10.

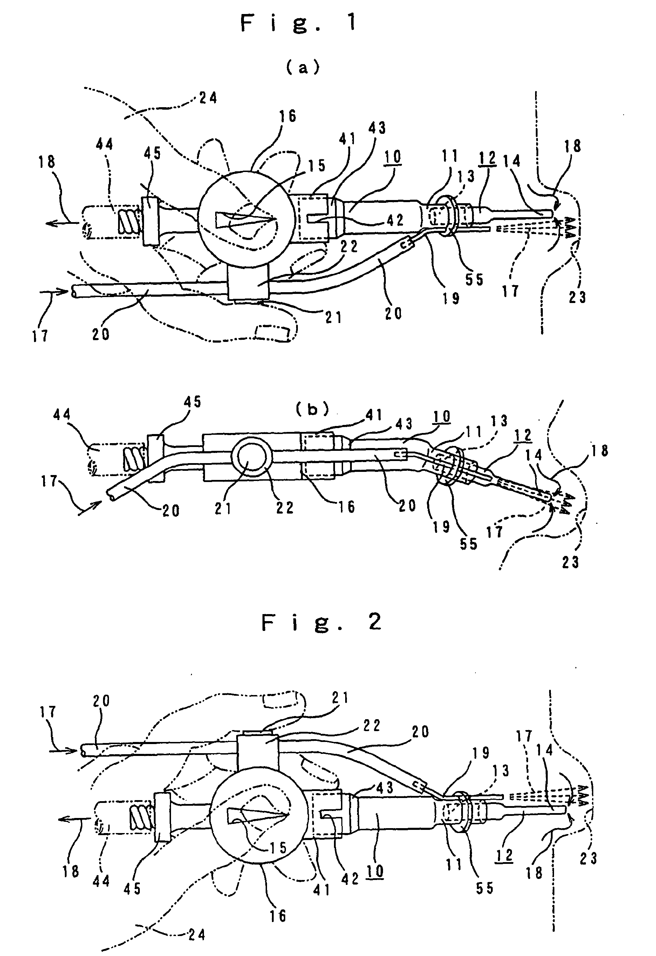

Next, to describe in detail of the condition of the waste matter sucking tube 44 and the condition of the front end portion of the rinsing liquid tube 20 referring to Fig. 1 (a) and (b), the waste matter sucking tube 44 is connected with one end of the tube connector 45 of the sucking control chamber 16, while the rostral sucking nozzle 12 for drawing the waste water and the waste matter resulting from the rinsing operation is detachably connected with the other connecting base 41 of the sucking control chamber 16 through the interposed connection tube 10. The connection tube 10 may be straight or curved at an angle from 20 to 30 degrees if necessary for the efficient functioning of the insertion port 11. The insertion port 11 permits the detachable insertion of a connector 13 of the replaceable rostral sucking nozzle 12, and the front portion 14 of the rostral sucking nozzle 12 can be set facing the affected part 23.

The jet nozzle 19, set for orienting the same direction as that of the insertion port 11, is integrally attached to the side of the insertion port 11 by being held securely with a silicon olive tube holder 55, while the basal end portion of the jet nozzle 19 is connected with the rinsing liquid tube 20.

Dimensionally, for example, the connection tube 10 is 8 mm in diameter; the jet nozzle 19 is 2 mm in diameter; the length from the insertion end 43 to the front end of the jet nozzle 19 is 50 mm; the length the extended portion of the jet nozzle 19 from the front end of the connection tube 10 is 2 mm; the length from the holder 55 to the jet nozzle 19 is 17 mm.

The length L and the diameter D of the rostral sucking nozzle 12 vary depending on the depth and the width of the affected part 23 or the uses thereof; when the nozzle is to be applied for ear, nose or throat disease treatment, however, the nozzle is supposed to be about 40-80mm in length L and about 5-10 mm in diameter D.

Further, the rostral sucking nozzle 12 comprises the connector 13 and a front portion 14; the connector 13 is designed for removable insertion into the insertion port 11 of the connection tube 10 and is primarily defined according to the form of the insertion port 11. The insertion port 11 and the connector 13 are desired to be designed for permitting sure connection and easy disconnection; for instance, the both may be tapered slightly or the connector 13 may be provided with an intermediary outer peripheral projection for limiting the extent of insertion.

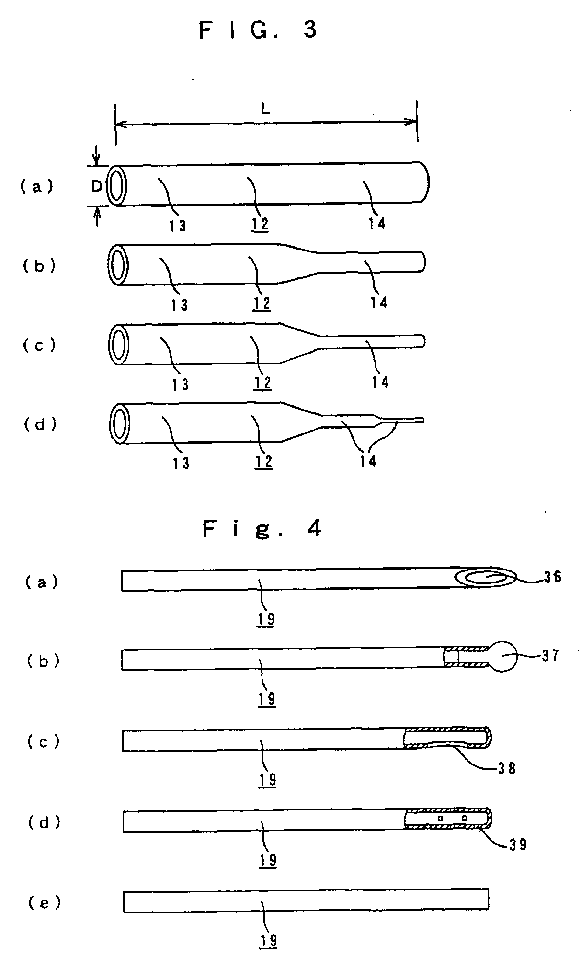

The form of the front portion 14 of the rostral sucking nozzle 12 is available in various types depending on the uses as shown in Fig. 3 (a) to (d).

In Fig. 3, (a) shows an embodiment, wherein the diameter of the connector 13 is identical with the diameter of the front portion 14; (b) shows an embodiment, wherein the diameter of the front portion 14 is about a half of the diameter of the connector 13; (c) shows an embodiment, wherein the diameter of the front end 14 is reduced to about a third to a quarter of the diameter of the connector 13; (d) is an embodiment, wherein the diameter of the front portion are divided into two portions having different diameters.

The rostral sucking nozzle 12 may be formed from a metal tube for repetitive use requiring the sterilization by boiling or may be formed from the plastic materials for disposable use. The types of the rostral sucking nozzle 12, however, are not limited to those discussed above.

As shown in Fig. 1, the form of the front end of the jet nozzle 19 is not limited to one formed by cutting a cylindrical tube at a right angle but may take various forms as are described in Fig. 4 (a) to (e).

In Fig. 4, (a) shows a case of a front portion 14 having an oblique opening 36 formed by obliquely cutting the end thereof just like the case of an needle for injection; this type of the front portion is used for the rinsing of a closed cavity such as the maxillary sinus or the like; (b) shows a case where the front end of the front portion 14 is filled with a porous material 37 such as the sponge; such front end is used for reducing the susceptibility to the injury of the part to be treated; (c) shows a case where a sidewise oblong opening is provided so that the rinsing liquid 17 can be jetted from the side of the front portion 14; (d) shows a case where a number of pinholes 39 are provided so that the rinsing liquid can be jetted from the circumference of the front portion; (e) shows a disposable front portion made from the plastics or the like.

Each of these jet nozzles 19 may be fixed to the side of the front portion of the connection tube 10 or detachably installed for permitting to be replaced when necessary. When using the detachable installation type jet nozzle 19, it is desired to employ the slip preventive type such as the screw type, the press-in type with O-ring or the like, since the effect of the pressurized rinsing water 17 need to be taken into consideration. Further, similarly to the case of the rostral sucking nozzle 12, each of these jet nozzles 19 may be formed from the metal tube for repetitive use requiring the sterilization by boiling or may be formed from the plastics as the one-time-use disposable type. Further, the jet nozzle 19 is not limited to the type described above.

The sucking control chamber 16 is designed to have a size suiting for being held with one hand 24 of an operator and a small-diameter cylindrical form. The front end of the sucking control chamber 16 is provided with a basal portion for a connecting base 41, provided with a slot 42 for permitting the detachable connection with the end portion for insertion 43 of the connection tube 10, and a tube connector 45, formed with the rear end the sucking control chamber 16, is connected with the waste matter sucking tube 44. Further, an air intake hole 15 provided with a push button 21, operable by the thumb action of an operator, is provided on one side of the sucking control chamber 16. The jet valve 22, provided with the push button 21, is installed on the external circumference of the sucking control chamber 16.

Further, the air intake hole 15 may be, besides the triangular opening as is shown in Fig. 1 and Fig. 2, an oblong slit-shape opening, a plurality of round openings having either identical or different diameters as are described later.

The jet valve 22 is, for instance, designed so that depression of the push button 21 against the internal spring of the cylindrical housing will cause the rinsing liquid tube 20 to open, while the release of the push button 21 will keep the rinsing liquid tube 20 closed by being pressed by means of the spring to interrupt the passage of the rinsing water 17.

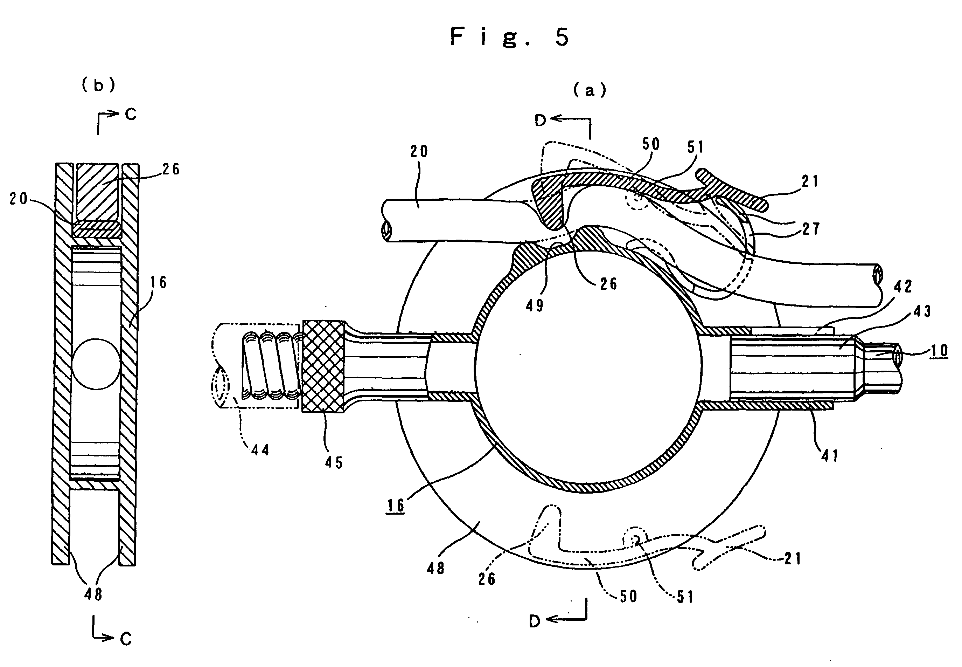

Fig. 5 (a) and (b) show the jet valve 22 as another embodiments, wherein a rinsing liquid tube 20, made from a soft material such as the silicon rubber, passes through the gap between the two collars 48 of the sucking control chamber 16; in the normal state, a swinging member 50, integrally formed with the push button 21 is pivotally supported with a fulcrum pin 51; a pusher 26 is forced downward by a leaf spring 27 to press the rinsing liquid tube 20 against a concave 49 to close the rinsing liquid tube 20 to thereby prevent the passage of the rinsing water 17. Pushing the push button 21 against a spring 27 will cause the rinsing liquid tube 20 to be opened to permit the passage of the pressurized rinsing liquid from the rinsing water tank 33.

The function of the medical rinsing and sucking device having the construction as is described in the foregoing will be described in the following.

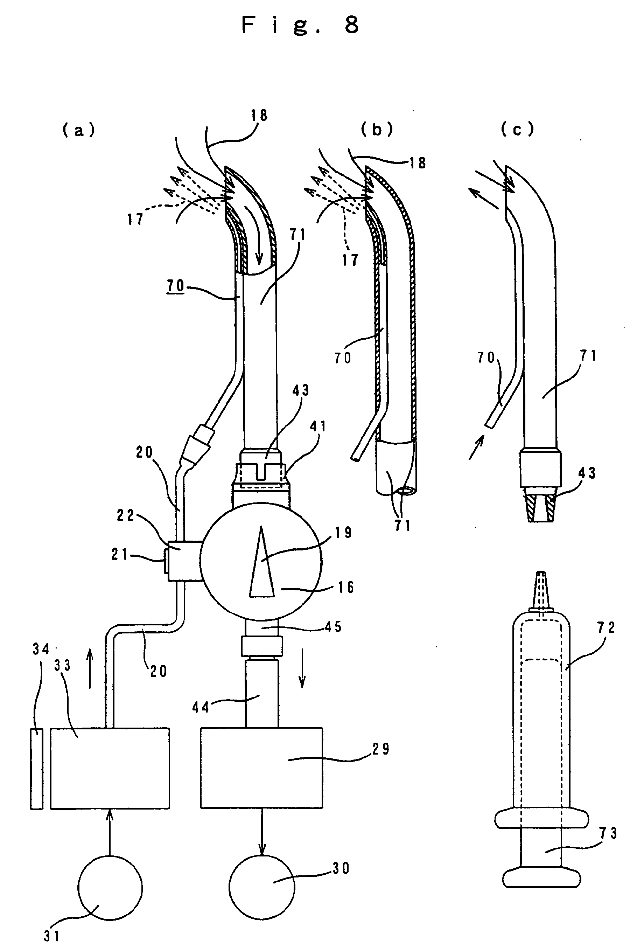

To operate the device, first turn on the power switch of the sucking pump 30 and the power switch of the compressor 31 in Fig. 8. When using the device primarily for rinsing the affected part of the out-patient, the sucking control chamber 16 is held with the right hand 24 while closing the air intake hole 15 with a thumb and lightly placing the first finger on the push button 21 of the jet valve 22. In this situation, first thing to be done is to insert the end portion for insertion 43 of the connection 10 into the basal portion for a connecting base 41, and the next thing to be done is to insert the rostral sucking tube 12 into the front portion of the connection tube 10. The rostral sucking tube 12 suiting the purpose of the use thereof is to be selected from among those having various forms as are shown in Fig. 3 prior to the insertion into the insertion port 11. The jet nozzle 19 suiting the purpose of the use thereof is to be selected from among those having various forms as are shown in Fig. 4.

The push button 21 is to be pushed after setting the front end of the rostral sucking nozzle 12 to face the affected part 23. This causes the pressure applied onto the rinsing liquid tube 20 by the spring 27 to be released, whereby the rinsing liquid tube 20 is made to open owing to the pressure of the pressurized rinsing water to thereby permit rinsing water 17 to be jetted against the affected part 23 from the front end of the jet nozzle 19 connected with the rinsing liquid tube 20. The jetted rinsing water, when the affected part 23 is indented as shown in Fig. 1, creates the convection current owing to the pressure of the rinsing water 17 to rins the affected part by the sterilization, the removal of the earwax, scab or the like. When the air intake hole 15 is kept open, little sucking is made from the front end of the rostral sucking nozzle 12, but, when the air intake hole 15 is closed with the thumb of the operator, the waste water 18 is drawn from the front end of the rotral sucking nozzle 12 to be recovered in the waste water tank 29 through the connection tube 10. The drawing from the rostral sucking tube 12 can be stopped or controlled by controlling the degree of the opening of the air intake hole 15 with the operator's thumb.

Thus, with the present device, the jetting of the rinsing water 17 and the sucking of the waste water 18 can be made simultaneously or alternately or selectively for either the jetting of the rinsing water 17 or the sucking of the waste matter such as the pus or blood by the single-handed operation of the operator.

Further, for the convenience of the operator who has to hold a surgical knife with the right hand or for the convenience of the rinsing operation by the left-handed operator, the device, as is shown in Fig. 2, wherein the location of the jet valve 22 or the location of the air intake hole 15 are reversed compared with those in the device for the right-hander, as is shown in Fig. 1, may be made available so that the device can also be used by the left-hander for the rinsing of the affected part 23.

In the embodiment shown in Fig. 6, the compressed air is directly introduced into the rinsing water 17 in the rinsing water tank 33 through the pressure tube 32 from the compressor 31. In such a event, sometimes it happens that the rinsing water 17 is contaminated to cause the infectious disease of the patient.

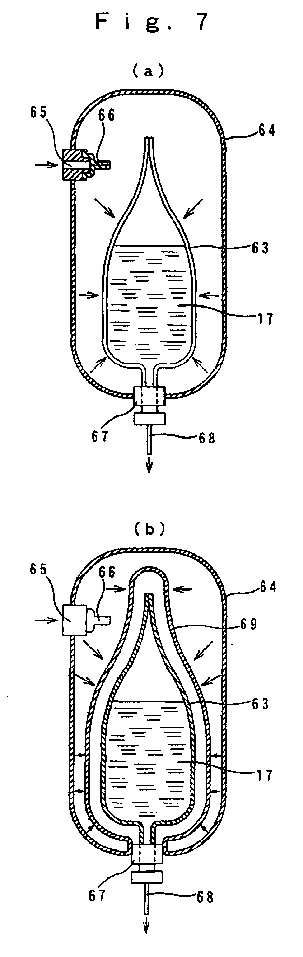

Thus, as shown in Fig. 7 (a), the rinsing water 17, previously stored in a sealed flexible storage bag 63, is contained in an outer casing 64, provided with a sealed valve 67, for airtight installation, while the compressed air is supplied from the compressor 31, connected with a pump connector 65, through a check valve 66 to pressurize the rinsing water 17 from the outside of the storage bag 63 to be jetted from a discharge tube 68.

Further, where it is difficult for the airtight mechanism for the connector of the sealed valve 67 for both the outer casing 64 and the storage bag 63 to be provided, another internal bag 69 may be airtighly provided inside the outer casing 64, thereby making unnecessary the airtight installation of the sealed valve 67 for the storage bag 63.

Fig. 8 (a) shows another embodiment of the present invention, wherein a jet needle 70 and a sucking needle 71, which are integrally fixed to each other and whose front portions are mildly curved while whose front ends are cut parallel to the axial lines thereof to form what is called the piercing needle ends respectively. Further, the basal portion of the sucking needle 71 is connected with the waste matter tank 29, while the basal portion of the rinsing liquid tube 20, connected with the jet needle 70, is connected with the rinsing water tank 33.

With such a construction of the present device, the piercing needle portions of the jet needle 70 and the sucking needle 71 are made thrust into the bone or the like for rinsing or the sucking of the waste water. Further, as in Fig. 1, the embodiment shown in Fig. 8 (a) is also provided with a sucking control chamber 16 and the jet valve 22 as intermediary constituents, and the descriptions thereof are omitted here, since such descriptions are similar to those given previously.

As shown in Fig. 8 (b), the jet needle 70 may be contained inside the sucking needle 71.

Further, as shown in Fig. 8 (c), an injection cylinder 72 to serve as a sucking pump may be connected with the end for insertion 43 of the sucking needle 71 to be substituted for the sucking control chamber 16 to draw the waste water or the waste matter resulting from the rinsing operation by the injection cylinder 72.

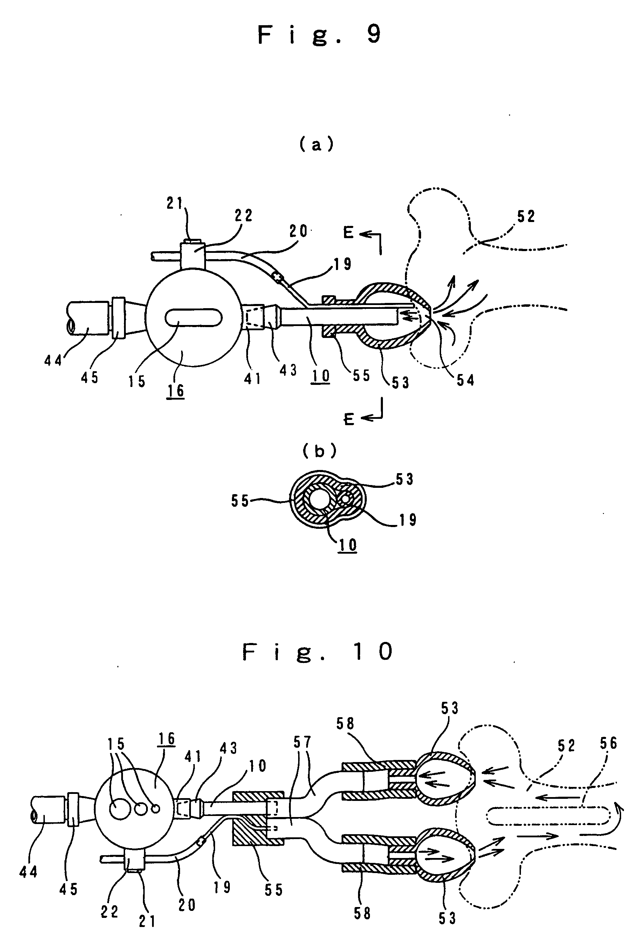

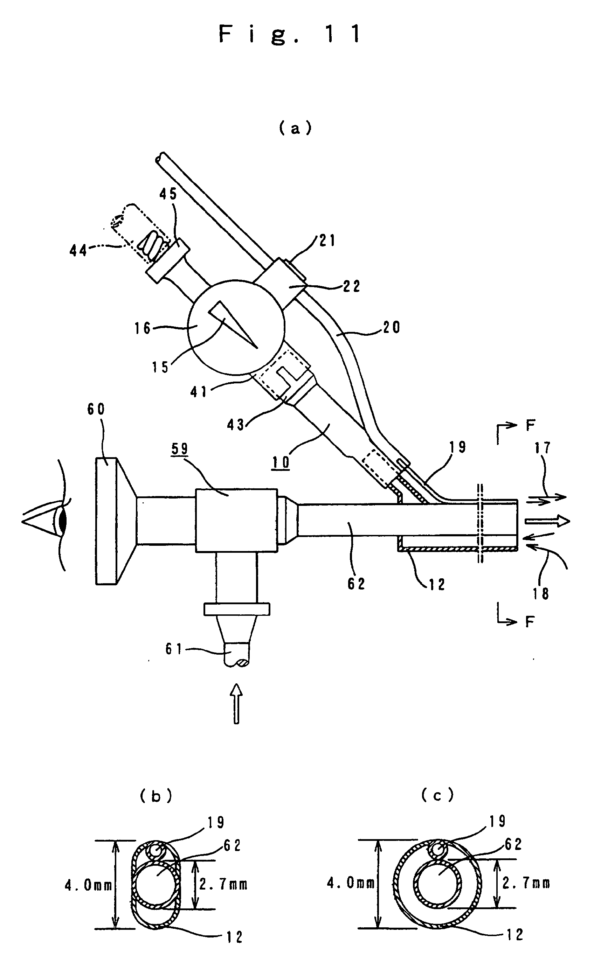

The embodiment shown in Fig. 1 is primarily designed for application to the treatment of the ear but may also be applied to the treatment of one nostril as shown in Fig. 9 and also for the treatment of two nostrils as shown in Fig. 10.

As shown in Fig. 9 (a) and (b), a nostril adapter 53 is set with both the front end of the connection tube 10, serving as a sucking tube too, and the front end of the jet nozzle 19. As shown in (b), since the section of the combination of the connection tube 10 and the jet nozzle 19 present a section formed with a section of a tube having a larger outside diameter and a section of a tube having a smaller outside diameter, it is desired for the nostril adapter to be made from a flexible material capable of tightly fitting the outside form of the combination of the connection tube 10 and the jet nozzle 19 or from a hard material to be previously formed to fit the outside of sectional form of the combination of the connection tube 10 and the jet nozzle 19.

In the case of the adapter for one nostril, the nostril adapter 53 is inserted into one of the nostrils of the nose 52 to carry out the rinsing and the sucking through the open end 54. To rins the two nostrils, the device is used alternately. Excessively pressurized rinsing water 17, when jetted from the jet nozzle 19, may reach too far in the nostril to give some pain to the patient, and thus the pressure of the rinsing water 17 needs to be adjusted so as not to reach too far or the sucking pressure of the connection tube 10 that serves as a sucking tube need to be set so that the rinsing water can be prevented from reaching too far by the effect of the sucking pressure.

In the embodiment for two nostrils shown in Fig. 10, the nostril adapters are fitted to the front end of the connection tube 10 to serve as a sucking tube too and the front end of the jet nozzle 19 respectively through a hard forked tube 57 and two soft flexible tubes 58. The soft flexible tubes 58 are employed to make the present device adaptable to the personal difference among different patients such as the difference in the interval between the two nostrils.

When using the double nostril adapter type device, first one nostril adapter 53 is to be inserted into one of the nostrils and then the other nostril adapter 53 is to be inserted into the other nostril. Then, the rinsing water 17 is jetted into one nostril while the waste water 18 resulting from the rinsing is drawn through the other nostril. In such a situation, when the pressure on the sucking side is set higher than the jetting side, the whole rinsing water 17 can be drawn passing the nasal septum 56. Thus, there hardly occurs that the rinsing water 17 jetted from the jet nozzle 19 reaches the depth of the nostril to cause a pain to the patient.

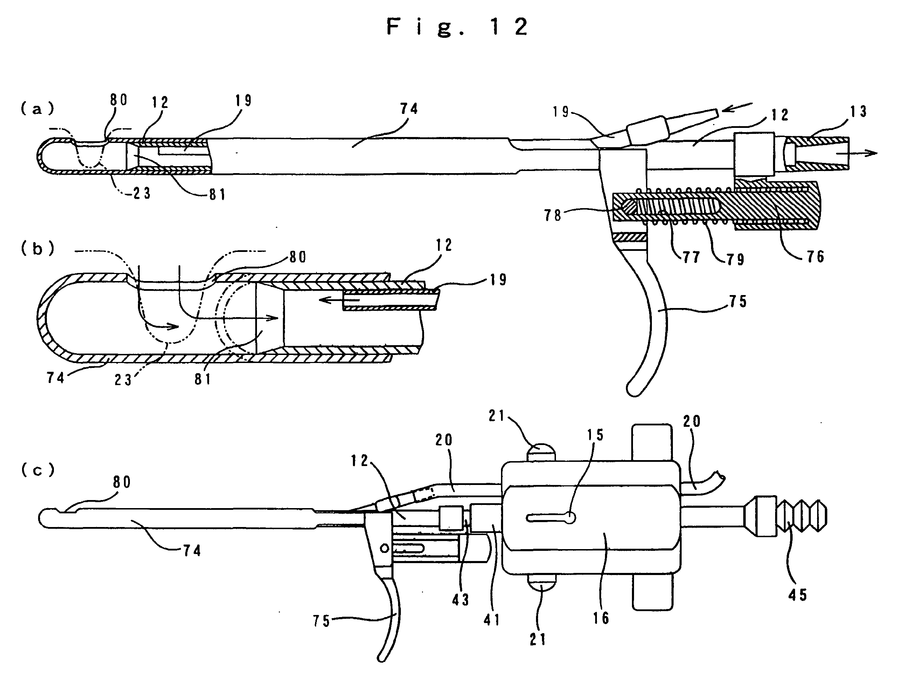

Fig. 11 shows another embodiment of the present invention, wherein the present medical rinsing and sucking device is combined with an nasal cavity endoscope 59. This nasal cavity endoscope 59 comprises a linear photoconductive rod 62, a lens container 60, to be looked into by an operator, provided on the side of the basal portion of the endoscope and a light cable 61, for supplying the light to the photoconductive rod 62, provided intersecting the photoconductive rod 62 at a right angle, while the rostral sucking nozzle 12 and the jet nozzle 19, respectively connected with the connection tube 10 and installed facing the front end of the photoconductive rod 62. The diameter of the photoconductive rod 62, which has been available to 4mm at the smallest, has now become available to 2.7 mm at the smallest, and so the space made available by reducing the diameter by 1.3 mm can be used for the installation of the rostral sucking nozzle 12 and the jet nozzle 19. For example, the jet nozzle 19, the sucking nozzle 12 and the photoconductive rod 62 may be arranged either to have an oval section having the major axis of 4 mm and the minor axis of 2.7 mm as represented by the embodiment shown in Fig. 11 (b) or to have a circular section of 4 mm in diameter including the section of the jet nozzle 19 and the section of the sucking nozzle 12 arranged outside the photoconductive rod 62 as represented by the embodiment given in Fig. 11 (c).

With such a construction, the operator not only can simultaneously carry out the treatment and the rinsing of the affected part while observing the affected part with an nasal cavity endoscope 59 but also can rins to prevent the front end of the photoconductive rod 62 from becoming cloudy unlike the case of the photoconductive rod 62 used in the conventional device.

Fig. 12 shows an embodiment of the present invention relating to the medical rinsing and sucking device whose front end is provided with a mechanism designed for excising and recovering an protrusion of the affected part 23 together with the rinsing liquid.

More specifically, as shown in Fig. 12 (a) and (b), a sliding cylinder 74 is slidably fit to the outside of the rostral sucking nozzle 12, while a sucking hole 80 is provided at the side of the rostral sucking nozzle 12 away by a certain distance towards the side of the operator from the closed front end of the sliding cylinder 74. Further, a sharp blade 81 is provided along the whole circumference of the front end of the rostral sucking nozzle 12. Further, a trigger 75 for starting the excising operation is integrally provided with the basal end of the sliding cylinder 74; a stopping projection 78 formed with the trigger 75 fit in an oblong hole 77 of a guiding rod 76, causing the sliding cylinder 74 to be always pushed forward by a coil spring 79.

With this construction, as shown in Fig. 12 (c), when the air intake hole 15 of the sucking control chamber 16 is closed with a finger to let a small protrusion of the affected part 23 be drawn towards the sucking hole 80 of the sliding cylinder 74, and the trigger 75 is pulled with a finger against the force of the coil spring 79, the protrusion is excised by being pinched between the internal circumference of the sucking hole 80 and the blade 81 of the rostral sucking nozzle 12 as shown in Fig. 12 (b). The excised protrusion is drawn together with the liquid used for rinsing to be wasted by operating the push button 21 of the sucking control chamber 16.

In the above embodiment, since the sliding cylinder 74 is designed for permitting the sliding motion thereof, the jet nozzle 19 for the rinsing liquid is desired to installed inside the rostral sucking nozzle 12 as shown in Fig. 12(b).

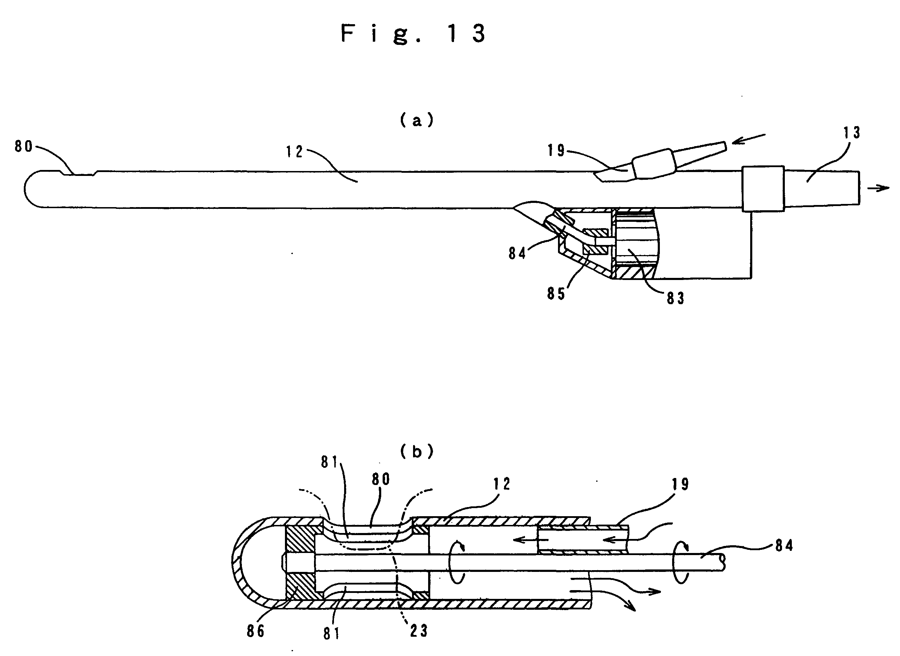

Fig. 13 shows another embodiment designed for excising the protrusion of the affected part 23. In the embodiment shown in Fig. 12, the sliding cylinder 74 is provided outside the rostral sucking nozzle 12 so that the protrusion of the affected part 23 is excised by the sliding motion of the sliding cylinder 74. However, in the embodiment shown in Fig. 13, the front end of the rostral sucking nozzle 12 is closed; the sucking hole 80 is provided at a location on the rostral sucking nozzle 12 closer by a certain distance towards the basal portion thereof; a rotatable cutter blade 86 is installed inside the rostral sucking nozzle 12 at the location facing the sucking hole 80; the blade 81 is formed with the cutter blade 86; the cutter blade 86 is connected with a flexible shaft 84; the flexible shaft 84 is connected with the motor 83, provided with the basal portion of the rostral sucking nozzle 12, through the connecting member 85.

With this construction, the protrusion of the affected part 23 is excised by rotating the cutter blade 86 while the protrusion is being pneumatically drawn into the sucking hole 80. The excised protrusion is drawn together with the rinsing liquid for disposal.

Further, the jet nozzle 19 may be installed on either the inside or the outside of the rostral sucking nozzle 12, since the rostral sucking nozzle will not be moved.

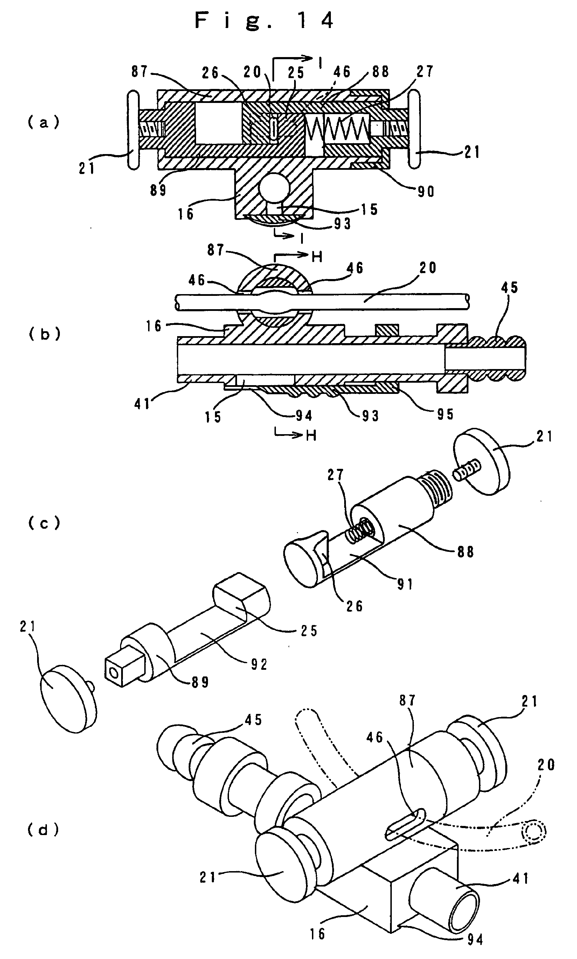

Fig. 14 shows another embodiment of the medical rinsing and sucking device according to the present invention. In this embodiment, the sucking control chamber 16 comprises a rectangular parallelepiped with the connecting base 41 provided at the rear end thereof and the tube connector 45 provided at the front end thereof, a cylindrical member 87, provided integrally with and orthogonally to the top of the rectangular parallelepiped, the oblong through holes 46, provided between the corresponding middle points on the front side and rear side of the cylindrical member 87, and the first pushing member 88 and the second pushing member 89 to function in combination with each other are inserted into the inside of the cylindrical member 87 having the open ends at both ends thereof. More specifically, as shown in Fig. 14 (c), a fitting concave 91 for the first pushing member 88 and a fitting concave 92 for the second pushing member 89 are provided respectively, and either the first pushing member 88 or the second pushing member 89 can be axially turned over so that the fist pushing member 88 and the second pushing member 89 can be fit to each other, while keeping the pusher 26 of the first pushing member 88 in close contact with a stationary part 25 of the second pushing member 89 by means of the spring 27 built in the first pushing member 88, when to be inserted from one side of the cylindrical member 87 and then to be fixed with a cover 90. In this way, the end portion of the second pushing member 89 and the end portion of the first pushing member 88 project respectively from the both ends of the cylindrical member 87, and the push button 21 is driven into each of the projected end portions. When one or both of the push buttons on the ends of the pushing members is or are pushed against the spring 27, this action causes a gap to be formed between the pusher 26 and the stationary part 25 so that the rinsing liquid tube 20 can be inserted into the gap from the through hole 46. After inserting the pusher members, when the push to the push button 21 is released, the pusher 26 and the stationary part 25 come into close contact to close the rinsing liquid tube 20. Pushing and the release of the push button 21 effect the close and opening of the rinsing liquid tube 20.

Further, an air adjusting cover 93 is slidably fit to a sliding groove 94, provided facing the air intake hole 15 at the bottom of the sucking control chamber 16, and a ring 95, provided integrally with the air adjusting cover 93, fits the cylindrical member of the sucking control chamber 16. The adjustment of the opening degree of the air intake hole 15 by the air adjusting cover 93 can be substituted for the adjustment by the operator's finger.

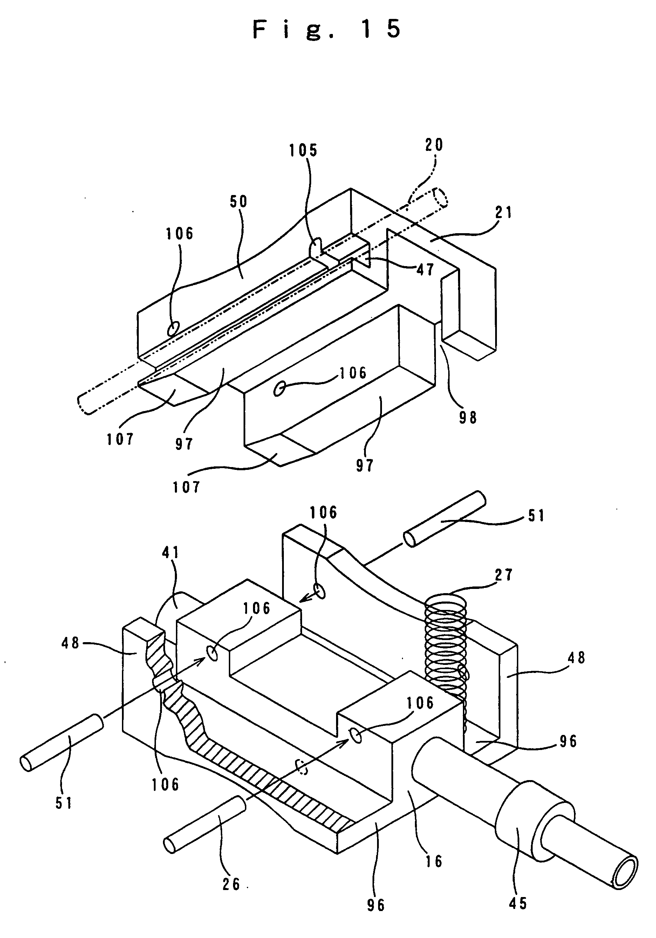

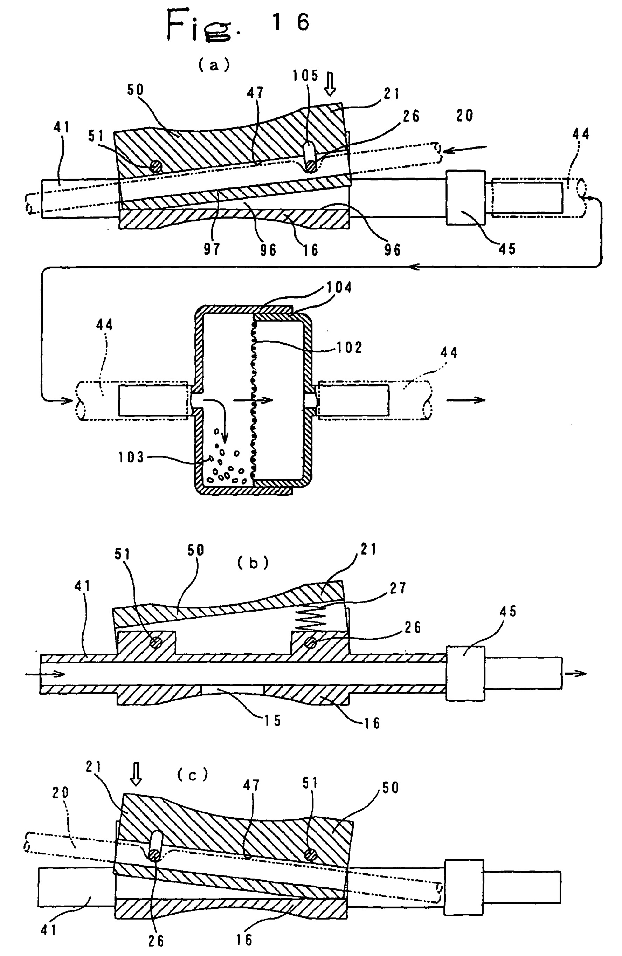

Fig. 15 and Fig. 16 show another embodiment of the present invention relating to the medical rinsing and sucking device respectively. In the embodiment shown in Fig. 15, the sucking control chamber 16 is formed into E-shape having the open side thereof facing upward; the connecting base 41 and the tube connector 45 are connected with the two ends of centrally provided sucking hole; a swinging grooves 96 is formed between the central portion and the collars 48 on both sides respectively. A swinging member 50, fitting to the sucking control chamber 16 is formed into a square U-shape opening downward to provide the engaging parts 97 facing downward on both sides thereof; one engaging part 97 has the through hole 47 formed passing therethrough sidewise, while the other engaging part 97 has a spring storage room formed from underside. Further, the engaging part 97 of the swinging member 50 is fit into the swinging groove 96 of the sucking control chamber 16 together with an interposed spring 27; the pin 51 is inserted into a shaft hole 106 provided at the front of the chamber, and the pin to serve as the pusher 26 is inserted into a shaft hole 106 provided at the rear portion of the chamber and an oblong hole 105. Then, as shown in Fig. 16 (a), the rear portion of the swinging member 50 is raised by the spring 27, so that, when the raised rear portion 50, to serve as the push button 21, is pressed within the oblong hole 105 against the force of the spring 27, the pusher 26 is made to be positioned at the upper part of the oblong hole 105 to open the through hole 47 thereby permitting the insertion of the rinsing liquid tube 20. When the depression of the push button is released, the rinsing liquid tube 20 is pressed between the pusher 26 and the bottom of the through hole 47 to be closed tightly. Further, the bottom of one end of the engaging part 97 is provided with a tapered surface 107 to smooth the up and down motion of the swinging part 50.

In the embodiment shown in Fig. 16 (a), the pin to serve as the pusher 26 in the swinging part 50 is provided on the side of the tube connector 45 of the sucking control chamber 16; however, as shown in Fig. 16 (c), the fitting position of the swinging part 50 may be reversed, and the position of the pin to serve as the pusher 26 may be located on the side of the connecting base 41 of the sucking control chamber 16.

In the embodiment shown in Fig. 16 (a), an intermediary container 104, provided with a filter 102, may be installed on the side of the tube connector 45 of the sucking control chamber 16 so that the waste matter 103 included in the waste water resulting from the rinsing operation can be removed too.

In the above embodiment, the opening of the air intake hole 15 of the sucking control chamber 16 is adjusted with the finger of the operator to adjust the rate of sucking, while adjusting the jetting rate of the rinsing liquid to be jetted from the jet nozzle 19 by controlling the push button 21 by the first finger of the operator.

Further, the air intake hole 15 and the push button 21 may be provided side by side so that both the sucking rate and the jetting rate of the rinsing liquid can be controlled only with the thumb action of the operator.

Further, the present device can be set differently so that, when the air intake hole 15 is not closed with the operator's thumb, that is, when the sucking is not being made, the rinsing liquid will not be jetted, or when the air intake hole 15 is closed with the thumb, that is, only when the sucking is being made, the jetting of the rinsing liquid can be controlled, or inversely when the air intake hole 15 is closed with the thumb, that is, when the sucking is being made, the jetting of the rinsing liquid is not controlled, or when the air intake hole 15 is not closed with the thumb, that is, when the sucking is being made, the jetting of the rinsing liquid is controlled; when the air intake hole 15 is not closed with the thumb, that is, when the sucking is not being made, the jetting of the rinsing liquid can be controlled.

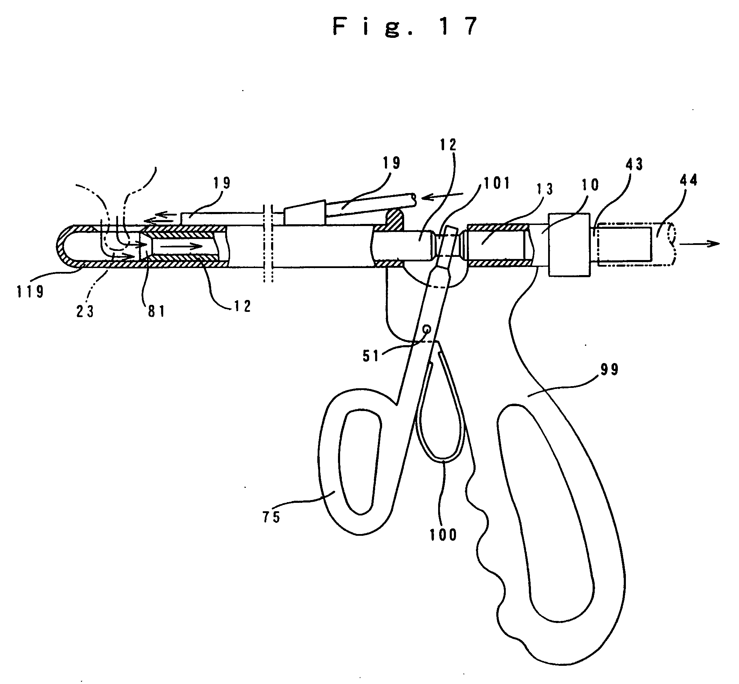

Fig. 17 shows further another embodiment of the present invention relating to the medical rinsing and sucking device. In the embodiment shown in Fig. 12, the sliding cylinder 74 is employed, so that there is the possibility that the protrusion excised from the affected part 23 and drawn into the sucking hole 80 of the sliding cylinder 74 gets out of the sucking hole 80 while the sliding cylinder 74 is in motion. Thus, in the embodiment shown in Fig. 17, an outer cylinder 119 is made stationary while the rostral sucking nozzle 12 is made slidable; a grip 99 is stationarily fixed to the outer cylinder 119 and the connection tube 10; the front portion of the rostral sucking tube 12 is inserted into the outer cylinder 119; the connector 13 is inserted into the connection tube 10, so that, when the trigger 75 is pressed, while the front portion of the rostral sucking nozzle 12 is inserted into the connection tube 10, the rostral sucking nozzle 12 in the outer cylinder 119 is made to project outside to excise the protrusion of the affected part 23.

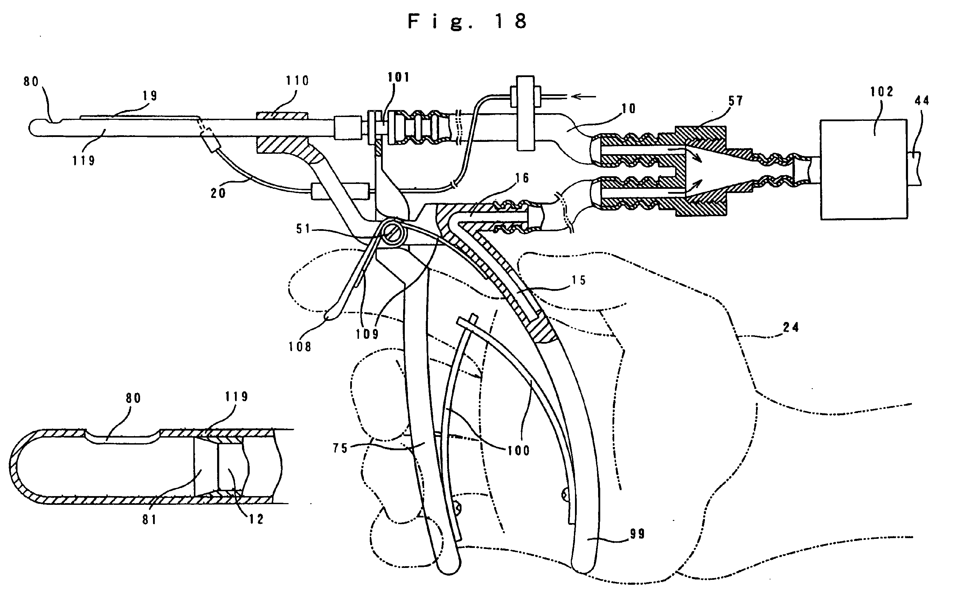

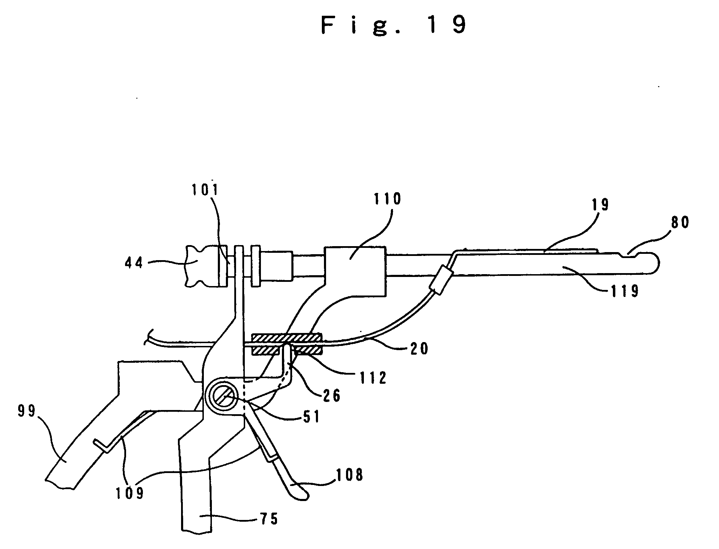

Each of Fig. 18 and Fig. 19 shows further another embodiment of the present invention relating to the medical rinsing and sucking device. In this embodiment, a grip 99 and a trigger 75 for excising operation are arranged crosswise each other and pivotally supported with a fulcrum pin 51 and an intermediary spring 100; another trigger 108 for the jetting of the rinsing water is pivotally supported with the fulcrum pin 51; a spring 109 is applied to the pin 51; the pusher 26, integrally formed with the trigger 108, is forced into the notch 112 of the tube holder 111 to press the rinsing liquid tube 20 to stop the supply of the rinsing liquid.

With the construction described above, the operator holds the grip 99 and the trigger 75 with one hand 24 as if holding a pair of scissors while hooking another trigger 108 with the first finger and placing the thumb on the air intake hole 15 provided in the grip 99. Then, when the trigger 108 is pulled with the index finger against the force of the spring 109, the rinsing liquid tube 20 is released to jet the rinsing liquid from the front end of the jet nozzle 19. The rinsing liquid, used for rinsing the affected part 23, is drawn into the internally installed rostral sucking nozzle 12 through the sucking hole 80 at the front end of the cylinder 74 to be discharged through the forked tube 57 and the filter 102. In this case, the sucking rate of the rinsing liquid used for the rinsing of the affected part can be controlled by adjusting the degree of opening of the air intake hole with the thumb.

When the trigger 75 is pulled with three fingers against the force of the spring 100, the rostral sucking nozzle 12, engaging with an engaging member 101, is made to project frontward, thereby causing a portion of the affected part, which has been drawn by the sucking force, to be excised by the inner circumferential edge of the sucking hole 80 and the blade 81 of the rostral sucking nozzle 12 and to be drawn together with the rinsing liquid. When the grip of the trigger 75 is eased, the rostral sucking nozzle 12 returns to the original position by means of the spring 100.

In this construction, the affected part is induced for being sucked into the sucking hole 80 to be excised with the blade 81, and simultaneously the open wound is rinsed with the rinsing liquid such as the physiological saline solution and the bleeding therefrom is stopped.

In the embodiment shown in Fig. 13, the front end of the rostral sucking nozzle 12 is closed; the sucking hole 80 is provided at a location somewhat nearer to the side of the operator; the cutter blade 86 is installed inside the rostral sucking tube 12 facing the sucking hole 80; the blade 81 is formed with the cutter blade 86; the cutter blade 86 is connected with the motor 83 through the flexible shaft 84. This embodiment is somewhat inconvenient in setting the position for the excision of the affected part 23 because of that the sucking hole 80 is provided at a location a little shifted towards the side of the operator on the rostral sucking tube 12.

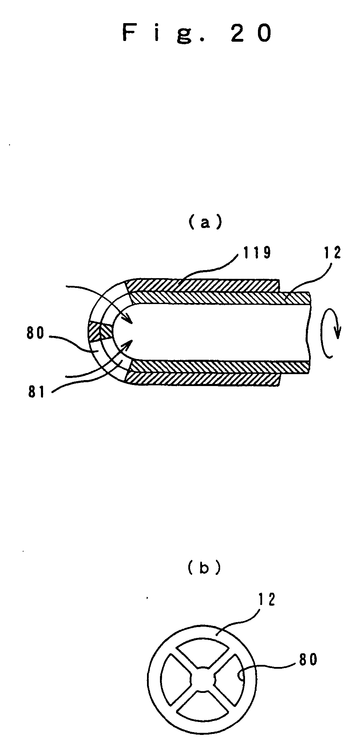

Each of Fig. 20 (a) and (b) shows another embodiment, wherein the position for excising the affected part is shifted to the front end of the rostral sucking tube 12. In this embodiment, a plurality of sucking holes 80 are formed at the front end of the outer cylinder 119, and there is provided the cutter blade 86 to rotate while maintaining the contact with the internal surface of the nozzle including the sucking hole 80. With this construction, while the protrusion of the affected part 23 is kept drawn into the sucking hole 80 of the outer cylinder 119, the blade of the cutter blade 86 is rotated by the motor 83 to excise the protrusion by the action of the cutter blade 86 like that of the electric shaver. The excised protrusion is collected together with the rinsing liquid to be discharged.

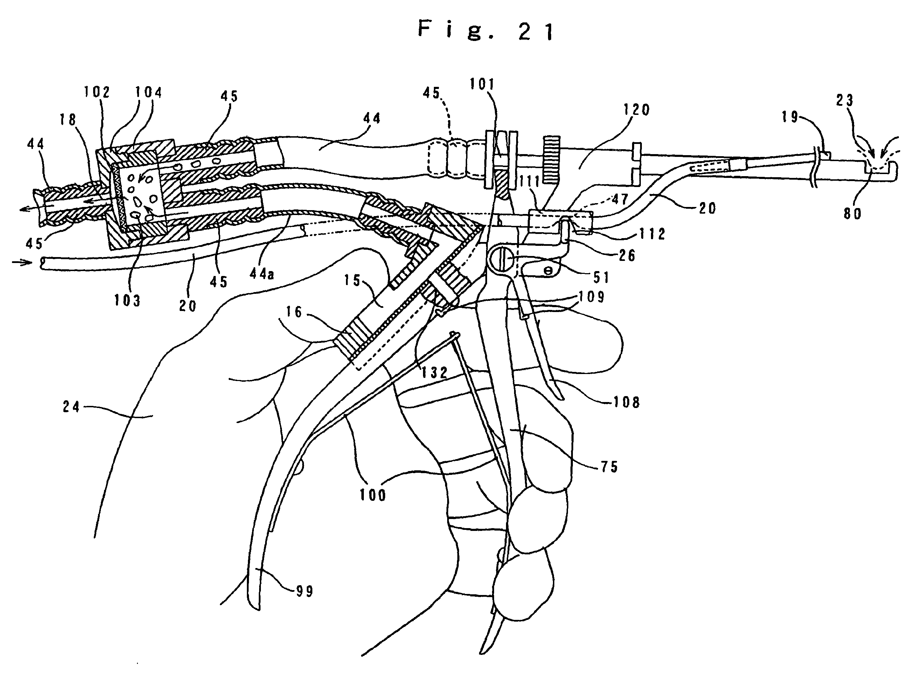

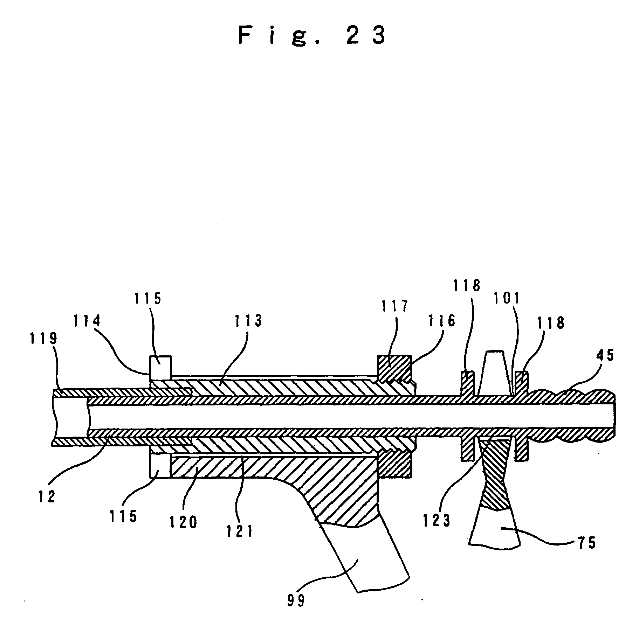

The embodiments shown in Fig. 21 to Fig. 24 are those improved from the embodiments given in Fig. 18 and Fig. 19.

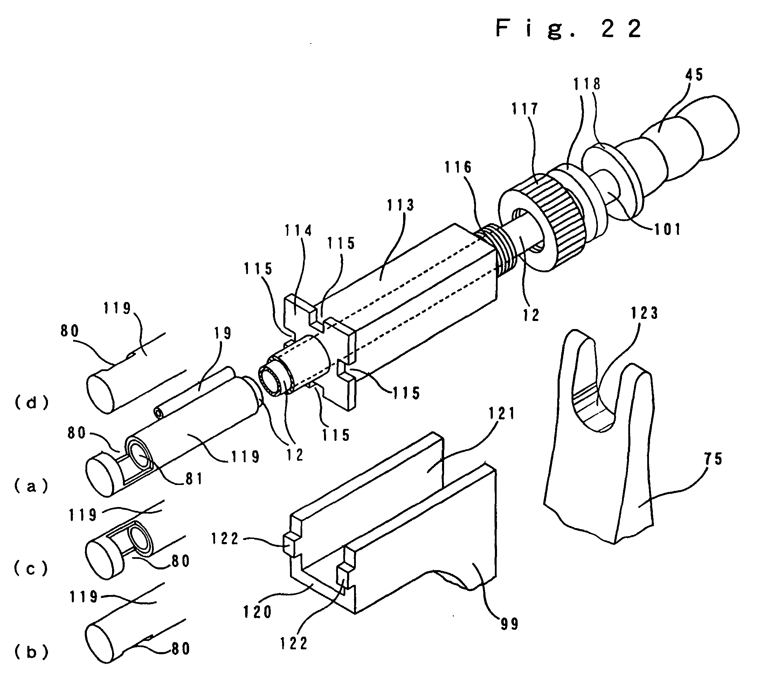

In Fig. 21, the grip 99 and the trigger 75 for excising operation are arranged crosswise each other and pivotally supported with the fulcrum pin 51; a supporting base 120 is integrally formed with the top end of the grip 99; the supporting base 120 includes a horizontally extending square fitting groove 121 opening upward as is shown in Fig. 22. Slippage preventive projections 122 are formed integrally with the two front edges of the supporting base 120.

Further, a tube holder 111, provided a little under the supporting base 120, is disposed horizontally like the corresponding one shown in Fig. 19. The tube holder 111 is provided with a through hole 47 for passing the rinsing liquid tube 20; a notch 112 is formed ranging from the bottom of the tube holder 111 to the through hole 47; the pusher 26, integrally formed with the trigger 108 for the rinsing water jetting operation, is forced into the notch 112 by means of the spring 109 to press and close the rinsing liquid tube 20.

As shown in Fig. 21, Fig. 22 and Fig. 23, an engaging concave 123 to engage with an engaging part 101, forming a part of the rostral sucking tube 12 which will be described later, is formed with the top of the trigger 75 for the excising operation.

As shown in Fig. 22, a square cylinder 113, designed to detachably fit in the fitting groove 121 formed with the supporting base 120, is provided; one end of the square cylinder 113 is integrally formed with an engaging stopper plate 114 being a little larger than the section of the square cylinder; the four sides of the engaging stopper plate 114 are provided with the slippage preventive notches 115 designed for permitting the engagement and disengagement with the slippage preventive projections 122; the circular outer cylinder 119 for guiding is integrally fixed to the center of the engaging stopper plate 114. The front end of the outer cylinder 119 is closed forming a round surface for smooth insertion towards the affected part 23, and the sucking hole 80 is provided on one side of the outer cylinder 119. The other end of the square cylinder 113 is integrally formed with a threaded portion 116.

The jet nozzle 19 for jetting the rinsing liquid or the like is integrally formed with the outer circumference of the outer cylinder 119 by means of the welding or the like; the front end of the jet nozzle 19 is disposed facing the sucking hole 80 while the other end thereof is connected with the rinsing liquid tube 20; the rinsing liquid tube 20 passes through the tube holder 111 to be connected with the pressurized rinsing water tank 33 as shown in Fig. 6.

A round through hole having a constant diameter is provided between the threaded portion 116 to the sucking hole 80, passing through the square cylinder 113, the engaging stopper plate 114 and the outer cylinder 119; the rostral sucking tube 12 is inserted into the round through hole from the side of the threaded portion 116 in a fashion allowing the forward and the backward sliding movement thereof; the blade 81 at the front of the rostral sucking tube 12 is disposed to face the sucking hole 80.

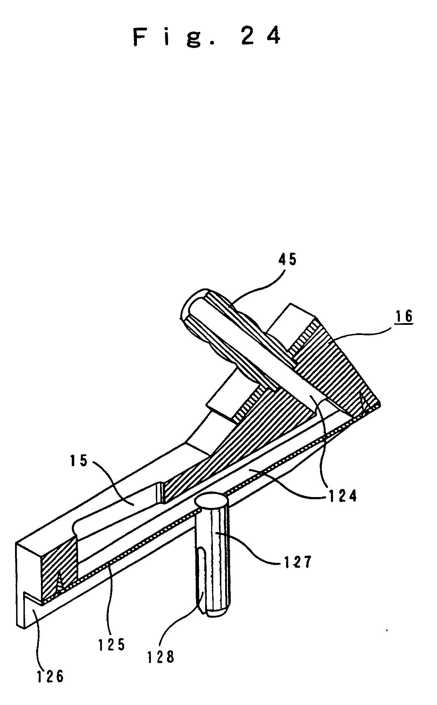

The grip 99 is detachably connected with the sucking control chamber 16 as shown in Fig. 21. As shown in Fig. 24, the sucking control chamber 16 is a little larger in width than the grip 99, provided with the air intake hole 15 opening upward and integrally formed with the tube connector 45, while the air intake hole 15 communicates with the tube connector 45 through a communication through hole 124 contained in a cover 125 and ranging from the air intake hole 15 to the tube connector 45. The sucking control chamber 16 has a fitting groove 126 running over the grip 99 formed with the bottom thereof. Further, the sucking control chamber 16 has a fixed pin 127 integrally formed with the bottom thereof and extending downward from about the center of the fitting groove 126. The slit 128 of the fixed pin 127 is opened slightly before being forced into the fixing hole 123 of the grip 99.

As shown in Fig. 21, the waste matter sucking tube 44 is connected with the tube connector 45 of the rostral sucking tube 12, while a sucking force control tube 44a is connected with the tube connector 45 of the sucking control chamber 16. The other ends of the waste matter sucking tube 44 and the sucking control tube 44a are connected with the forked tube connector 45 of the filtering container 104. The filtering container 104 contains the filter 102 and closed with a detachable threaded cover, while another tube connector 45 provided at the other end of the filtering container 104 is connected with the waste matter tank 29 as is shown in Fig. 6.

Next, prior to the use of the medical rinsing and sucking device according to the present invention, a rostral sucking nozzle 12 suiting the treatment of the affected part 23 needs to be chosen. After the nut 117 has been unscrewed, the front end of the chosen rostral sucking nozzle 12 is to be inserted into the square cylinder 113 from the side of the threaded portion 116. When the insertion of the rostral sucking nozzle 12 is completed, the square cylinder 113 is made to fit in the fitting groove 121 of the supporting base 120, while the engaging part 101 is made to engage the engaging concave 123.

At this stage, depending on the direction (e.g., upward, downward, rightward or leftward) of the affected part 23 to be excised and/or rinsed, the angle of the sucking hole 80 can be altered by 90 degrees to be set to any one of the directions (a), (b), (c) and (d) by being fit in the fitting groove 121 as shown in Fig. 22.

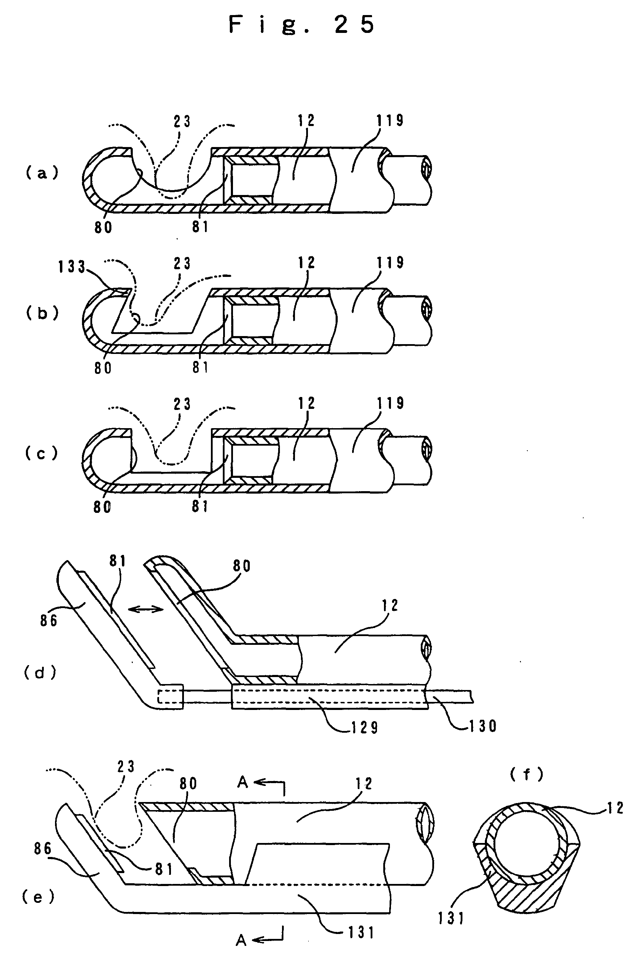

The square cylinder 113 is fitted in the fitting groove 121; the engaging part 101 is made to engage the engaging concave 123; then, the square cylinder 113 is made to slide towards the side of the operator within the fitting groove 121 to fit the slippage preventive notch 115 and the slippage preventive projection 122 with each other; the nut 117 is driven around the threaded part 116 to fix the outer cylinder 119 to the grip 99. At this stage, the blade 81 at the front end of the rostral sucking nozzle 12 reaches the point immediately before the sucking hole 80 as shown in Fig. 25 (a).

In this stage, as shown in Fig. 21, the grip 99 and the trigger 75 are held by one hand of the operator while putting the first finger on the trigger and placing the thumb on the air intake hole 15 of the sucking control chamber 16. Then, the sucking hole 80 at the front portion of the rostral sucking nozzle 12 is brought to the point facing the affected part 23.

When rinsing or sterilizing the affected part 23, the trigger 108 is pulled with one's first finger against the force of the spring 109, thereby making the rinsing liquid tube 20 opened to jet the rinsing liquid from the front end of the jet nozzle 19. The liquid used for rinsing is drawn into the rostaral sucking nozzle 12 through the sucking hole 80 and discharged through the forked tube 57 and the filter 102. A this stage, the jetting rate of the sterilizing liquid can be controlled by adjusting the position of the pusher 26 by the extent of the pull of the trigger 108 with the finger. Further, the intake rate of the air can be controlled by adjusting the opening degree of the air intake hole 15 with the thumb, thereby controlling the sucking rate of the liquid used for the rinsing of the affected part 23.

Next, when a portion of the affected part 23 is to be excised, the portion to be excised is brought to the point facing the sucking hole 80, and the trigger 75 is pulled with three fingers against the force of the spring 100, whereby the rostral sucking nozzle 12, engaging with the engaging part 101, is made to project forward to make the desired portion of the affected part 23 excised by means of the internal circumferential edge of the sucking hole 80 and the blade 81 of the rostral sucking nozzle 12 and drawn together with the rinsing liquid. When the grip to the trigger 75 is eased, the rostral sucking nozzle 12 returns to its original position by means of the spring 100.

In this way, the portion of the affected part 23 is drawn into the sucking hole 80 to be excised with the blade 81, and simultaneously the open wound after excision is rinsed with the physiological saline solution, and the open wound is treated for stopping the bleeding.

The waste matter resulting from the excising operation is collected on the filter 102 of the filter container 104, and only the waste water is transferred into the waste matter tank 29.

The form of the outer cylinder 119 and the front end form of the rostral sucking nozzle 12 are available in a variety of types depending on the uses as shown in Fig. 25 (a) to (e).

The embodiment (a) is characterized by a circular sucking hole 80 of the outer cylinder 119. This type suits the excision of relatively soft affected part.

The embodiment (b) is characterized by a square sucking hole 80 of the outer cylinder 119 having one end indented at an acute angle to form an engaging part 113 having an acute angle. This type suits for application, as one having a slippage preventive part suiting the excision of a hard object such as the bone.

The embodiment (c) is characterized by a square sucking hole 80 of the outer cylinder 119 suiting for both the soft and the hard objects.

The embodiment (d) is characterized by a sucking hole 80 formed by cutting the front portion of the rostral sucking nozzle 12 bent at about 45 degrees along the direction of the bend, while a guiding cylinder 129 is stationarily installed to the side of the rostral sucking nozzle 12; a sliding shaft 130, designed to be slidable back and force, is installed inside a guide cylinder 129; the blade 81, designed for excising the affected part 23 in collaboration with the sucking hole 80 by changing their relative positions, is formed with the front end of the sliding shaft 130. In this embodiment, either the rostral sucking nozzle 12 or the blade 81 or the both may be designed slaidable back and forth.

The embodiment (e) is characterized by a sliding cover 131, designed to be freely salidable back and forth and installed covering the outside of the rostral sucking nozzle 12, and the blade 81 provided with the front end of the sliding cover 131. In this embodiment too, either the rostral sucking nozzle 12 or the blade 81 or the both may be designed to be slidable back and forth.

The embodiment (f) is identical with the embodiment (e) except being represented by the sectional view.

The above embodiments are designed varying depending on the uses; for example, the length L and the diameter D of the rostral sucking nozzle 12 vary depending on the depth and width of the route for reaching the affected part; for application to the treatment of the ear, the nose and the throat, the rostral sucking nozzle 12 is designed to have the length L ranging from about 40mm to 80mm and the diameter D ranging from about 5 mm to 10 mm.

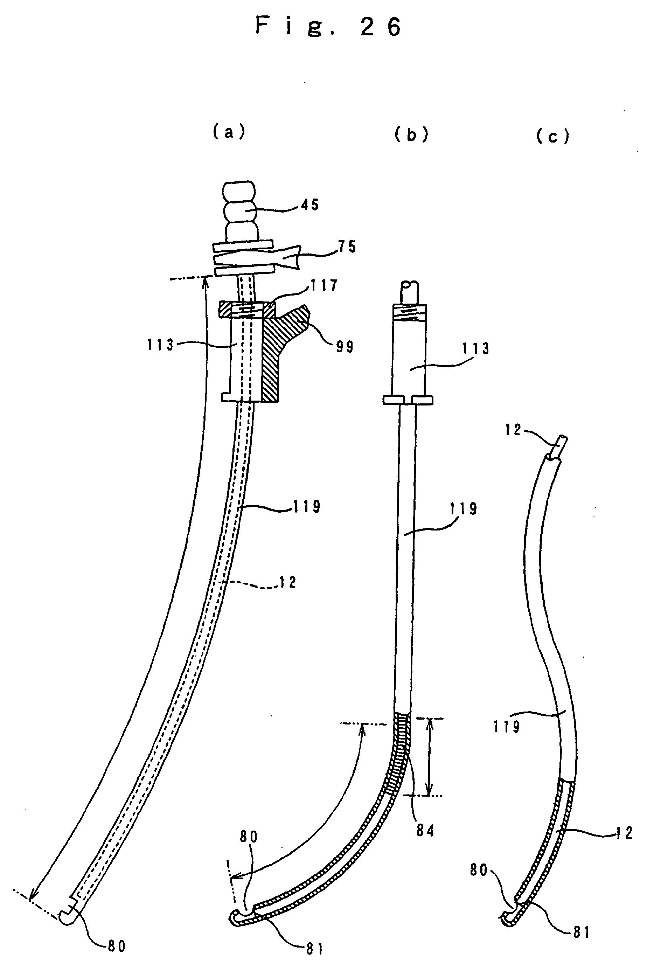

However, the device having a straight form is sometimes not suitable for the insertion into a human body. Thus, depending on the application, it is recommendable for the device to have curved forms as are shown in Fig. 26 (a), (b) and (c).

The embodiment (a) is characterized by the curved tube form of the rostral sucking nozzle 12 having a constant curvature ranging from the collar to the front end thereof. Thus, in order for this curved tube to be permitted to move back and forth inside the outer cylinder 119, it is necessary for the square cylinder 113, the engaging stopper plate 114 and the circular hole of the outer cylinder 119 to have a similar curvature.

The embodiment (b) is characterized by that a part of the rostral sucking nozzle 12, ranging from a specific point thereof to the front end thereof, is a curved tube having a specific curvature. In this embodiment, for the flexibility of the rostral sucking nozzle 12, the portion, including the point where the straight portion of the outer cylinder 119 starts to curve, is made flexible for permitting the free back-and-forth movement.

The embodiment (c) is characterized by that both the outer cylinder 119 and the rostral sucking nozzle 12 are made from the metal or plastic tubes having relatively small flexibility butt permitting to give desired curvature.

As described in the foregoing, the device according to the present invention permits the single-handed operation by an operator in carrying out the jetting of the rinsing water and the sucking of the waste water resulting from the rinsing either simultaneously or alternately or only the sucking of the pus, the blood or any other waste water.

### INDUSTRIAL APPLICABILITY

As discussed in the foregoing, the medical rinsing and sucking device according to the present invention permits the single-handed operation by an operator and the applications to the surgical operation ortreatments i nvolving the jetting of the rinsing liquid, the sucking of the waste water after the rinsing, the excision of the protrusion of the affected part, espe cially to treatments of those affected parts located in deep and narrow na sal cavity, the interior of the auditory organ and the throat.

## Claims

1. A medical rinsing and sucking device, comprising a connection tube 10, having a basal end portion connected with a waste matter sucking tube 44 communicating with a waste matter tank 29 provided with a sucking pump 30 and a front end portion connected with a rostral sucking nozzle 12 for drawing the waste water, and a jet nozzle 19 for jetting the rinsing water, having a basal end portion connected with a rinsing liquid tube 20 communicating with a rinsing water tank 33 provided with a compressor 31, wherein the said connection tube 10 and said jet nozzle 19 are installed making the respective front ends thereof orient substantially the identical directions.

2. The medical rinsing and sucking device according to claim 1, wherein a rostral sucking nozzle 12 is detachably connected with the front end of the connection tube 10.

3. A medical rinsing and sucking device, comprising a connection tube 10, having a basal end portion connected with a waste matter sucking tube 44 communicating with a waste matter tank 29 provided with a sucking pump 30 and a front end connected with a rostral sucking nozzle 12, and a jet nozzle 19, having a basal end connected with a rinsing liquid tube 20 connected with a rinsing water tank 33 provided with a compressor 31, wherein the connection tube 10 and the jet nozzle 19 are installed making the front ends thereof orient substantially the identical directions; a sucking control chamber 16 is interposed between said connection tube 10 and said waste matter sucking tube 44; an end portion for insertion 43 of said connection tube 10 is detachably connected with a basal portion for a connecting base 41 of the rostral sucking control chamber 16; an intermediary jet valve 22, provided with a push button 21 for opening and closing the rinsing liquid tube 20 connected with said jet nozzle 19, is provided.

4. The medical rinsing and sucking device according to claim 3, wherein the rinsing liquid 17 is made to be jetted by applying an external pressure to a sealed flexible bag 63 containing the rinsing water.

5. The medical rinsing and sucking device according to claim 3, wherein the jet valve 22 is designed to depress the rinsing liquid tube 20 by using the force of a spring 27 when to be closed and to open the rinsing liquid tube 20 by releasing the rinsing liquid tube 20 from the depression applied by the force of the spring 27.

6. A medical rinsing and sucking device, comprising a sucking needle 71 for the sucking of the waste matter, having a basal end portion connected with a waste matter sucking tube 44 communicating with a waste matter tank 29 provided with a sucking pump 30 and a front end portion formed into a piercing-needle form, and a jet needle 70 for jetting the rinsing liquid, having a basal end portion connected with a rinsing liquid tube 20 communicating with a rinsing water tank 31 provided with a compressor 31 and front end portion serving as a jet needle 70 for jetting the rinsing liquid formed into a piercing needle form, wherein the sucking needle 71 and the jet needle 70 are installed making the front portions thereof orient substantially the identical directions.

7. A medical rinsing and sucking device, comprising a connection tube 10, having a basal end portion connected with a waste matter sucking tube 44 communicating with a waste matter tank 29 provided with a sucking pump 30, and a jet nozzle 19 for jetting the rinsing liquid, having a basal end portion connected with a rinsing liquid tube 20 communicating with a rinsing water tank 33 provided with a compressor 31, wherein the front end of the connection tube 10 and the front end of jet nozzle 19 are provided with a common nostril adaptor 53, thereby enabling the rinsing water 17 to be jetted and the waste water 18 to be drawn through the open end of the nostril adapter 53.

8. A medical rinsing and sucking device, comprising a connection tube 10, having a basal end portion connected with a waste matter sucking tube 44 communicating with a waste matter tank 29 provided with a sucking pump 30 and a front end portion directed to the waste water sucking side, and a jet nozzle 19 for jetting the rinsing water connected with a rinsing liquid tube 20 communicating with a rinsing water tank 33 provided with a compressor 31, wherein the front end of the connection tube 10 and the front end of the jet nozzle 19 are respectively provided with a nostril adaptor, thereby enabling the rinsing water 17 to be jetted from opening 54 of the nostril adaptor 53 provided with the connection tube 10 and the waste water to be drawn from the opening 54 of the nostril adaptor 53 provided on the side of the connection tube 10.

9. A medical rinsing and sucking device, comprising a connection tube 10, having a basal end portion connected with a waste matter sucking tube 44 communicating with a waste matter tank 29 provided with a sucking pump 30 and a front end connected with a jet nozzle 19 for jetting the rinsing water, having a basal end portion connected with a rinsing liquid tube 20 communicating with a rinsing water tank 33 provided with a compressor 31, wherein the front end of the connection tube 10 and the front end of the jet nozzle 19 are respectively attached to the front end of a photoconductive rod of a nasal cavity endoscope 59 in a fashion permitting the front ends of the rostral sucking nozzle 12 and the jet nozzle 19 to face the front end of the photoconductive rod 62.

10. The medical rinsing and sucking device according to claim 1, wherein the waste matter tank 29, provided with the sucking pump 30, comprises an injection cylinder 72 and an injection piston 73.

11. The medical rinsing and sucking device according to claim 1, wherein a sliding cylinder 74 is fitted around the external circumference of the rostral sucking nozzle 12 so as to be slidable back and forth freely, whereby the protrusion of the affected part 23 can be excised or drawn by the relative motion between the sucking hole 80 at the front end of the sliding cylinder 74 and the back-and-forth movement of the blade 81 at the front end of said rostral sucking nozzle 12.

12. The medical rinsing and sucking device according to claim 3, comprising a sucking control chamber 16 sized for permitting one-handed grip, a centrally opening of air intake hole 15 for permitting the connection with the rostral sucking chamber 12, a through hole 46, ranging two sides of the rostral sucking nozzle 12 for permitting the insertion of a rinsing liquid tube 20, a swinging member 50 swingably installed to the outside of the rostral sucking nozzle 12 with a pin 51, a push button 21 integrally formed with one end of the a swinging member 50 and projecting out of the sucking control chamber 16, a pusher 26 provided with the other end of the swinging member 50 and projecting on the side of the rinsing liquid tube 20, the pusher 26 constituting a jet valve 22 that is constantly forced to close the rinsing liquid tube 20 by a spring 27.

13. The medical rinsing and sucking device according to claim 3, wherein the sucking hole 80 is provided a little closer to the operator from the closed front end of the rostral sucking nozzle 12; a rotatable cutter blade 86 is provided inside the rostral sucking nozzle 12 so as to face the location of the sucking hole 80; the cutter blade 86 is connected with a motor 83 through a flexible shaft 84 so that the protrusion of the affected part 23 can be excised by the rotary motion of the blade 81 of said cutter blade 86.

14. The medical rinsing and sucking device according to claim 3, wherein the sucking control chamber 16 comprises a rectangular parallelepiped, having a basal portion for a connecting base 41 at the front end thereof and a tube connector 45 at the rear end thereof, and a cylinder 87 integrally formed with the top of said rectangular parallelepiped at a substantially right angle thereto; a first pushing member 88 and a second pushing member 89, combined with each other by means of a corresponding open fitting concave 91 and a corresponding open fitting concave 92 by being aided by the spring 27 built in the first pushing member, are inserted into the cylinder 87 having open ends; the push button 21 is provided with each of the projecting portions of the first pushing member 88 and the second pushing member 89; the rinsing liquid tube 20 is inserted, passing the through hole 46 provided with said pushing member 87, into the gap formed between the pusher 26 of the first pushing member 88 and a stationay part 25 of the second pushing member 89 when one of push buttons 21 is pressed against the spring 27; the closing and the opening of the rinsing liquid tube 20 are effected by the depression and the release of the depression to the push button 21.

15. The medical rinsing and sucking device according to claim 3, wherein the sucking control chamber 16 has an E-shape opening upward; one end of a central sucking hole is connected with the basal portion for a connecting base 41 while the other end thereof is connected with the tube connector 45; a swinging grooves 96 are formed between the central portion and the collars 48 at two ends of the central portion; the swinging member 50 fitting the sucking control chamber 16 has a U-shape opening downward with both ends thereof provided with the engaging parts 97; one of the engaging part 97 is provided with a through hole 47, while the other end is provided with a spring containing space 98 opening downward; the engaging part 97 of the swinging member 50 is fit into the swinging groove 96 of the sucking control chamber 16 though an interposed spring 27; a pin 51 is inserted into one shaft hole 106, while the pusher 26 is inserted into the other shaft hole 106 and the oblong hole 105; the rear portion of the swinging member 50 is kept raised by the force of the spring 27 so that the rinsing liquid tube 20 can be closed when the push button 21 is pressed against the force of the spring 27 and opened when the push button 21 is released from depression.

16. The medical rinsing and sucking device according to claim 3, wherein a grip 99 is stationarily attached to a sliding cylinder 74 and the connection tube 10; the front end of the rostral sucking nozzle 12 is inserted into the sliding cylinder 74; a connector 13 is inserted into the connection tube 10; pulling a trigger 75, provided with the sliding cylinder 74, causes the rostral sucking nozzle 12 to move back and forth to excise the protrusion of the affected part 23 in collaboration between the sucking hole 80 provided at the front end of the sliding cylinder 74 and the blade 81 provided at the front end of the rostral sucking nozzle 12.

17. A medical rinsing and sucking device, comprising a connection tube 10, having a basal end portion connected with a waste matter sucking tube 44 communicating with a waste matter tank 29 provided with a sucking pump 30 and a front end connected with a rostral sucking nozzle 12 for drawing the waste water, and a jet nozzle 19, having a basal end portion connected with a rinsing liquid tube 20 communicating with a rinsing water tank 33 provided with a compressor 31, wherein the connection tube 10 and the jet nozzle 19 are installed in a fashion that the front ends thereof orient substantially the same directions; a sucking control chamber 16 is interposed between the connection tube 10 and the waste matter sucking tube 44; the connection tube 10 and the jet nozzle 19 are attached to the front end of the grip 99; said sucking control chamber 16 is disposed on the way to the grip 99; the trigger 75 for the rostral sucking nozzle 12 is pivotally installed with the grip 99 by means of a fulcrum pin 51; the fulcrum pin 51 also pivotally supports a trigger 108; the pusher 26, integrally formed with the trigger 108 for the jetting operates to open and close the rinsing liquid tube 20; the air intake hole 15 of the sucking control chamber 16 is provided with the grip 99.

18. The medical rinsing and sucking device according to claim 3, wherein a number of sucking holes 80 are formed with the front end of the cylinder 74; a cutter blade 86, designed to rotate in contact with the internal surface of the sucking hole 80, is provided with the front end of the rostral sucking nozzle 12; the apohysis of the affected part being kept drawn into the sucking hole 80 is excised by the rotary cutter 83 driven by a motor 83.

19. A medical rinsing and sucking device, wherein the rostral sucking nozzle 12 has a basal end portion, connected with a waste matter sucking tube 44 communicating with a waste matter tank 29 provided with a sucking pump 30, and a front end, serving for the sucking of the waste water and formed with the blade 81 for excising the affected part; the rinsing water jet nozzle 19 has a basal end portion connected with a rinsing water tank 33 provided with a compressor 31; a grip 99 and a trigger 75 for excising operation are arranged crosswise with each other or in an X-form arrangement; a fitting groove 121 is formed with a supporting base 120 provided with the top end of the grip 99; an engaging concave 123, for engaging with a part of the engaging member of the rostral sucking nozzle 12, is formed with top end of said trigger 75 for excising operation; a square cylinder 113 is detachably fit into said fitting groove 121; an external cyinder 119 for guiding is integrally formed with one end side of the square cylinder 113; the sucking hole 80, facing one side, is provided with the front end of the outer cylinder 119; the jet nozzle 19 is integrally attached to outer circumference of the outer cylinder 119; the front end of the jet nozzle 19 is set to face the sucking hole 80; the rostral sucking nozzle 12 is inserted, so as to be movable back and forth, into a circular hole ranging from the square cylinder 113 to the outer cylinder 119; the blade 81 at the front end of the rostral sucking nozzle 12 is set to face the sucking hole 80 of said outer cylinder 119; said grip 99 is provided with the sucking control chamber 16.

20. The medical rinsing and sucking device according to claim 19, wherein the outer cylinder 119 for guiding and the rostral sucking nozzle 12 designed for sliding back and forth inside the outer cylinder 119 formed into curved tubes..

21. The medical rinsing and sucking device according to claim 19, wherein the sucking hole 80 of the outer cylinder 119 for guiding is set to orient any desired direction when being fit into the fitting groove 121 of the supporting base 120.
